(19) Europäisches Patentamt
European Patent Office
Office européen des brevets

(11) Veröffentlichungsnummer : **0 433 988 B1**

# (12) EUROPÄISCHE PATENTSCHRIFT

(45) Veröffentlichungstag der Patentschrift : **10.11.93 Patentblatt 93/45**

(51) Int. Cl.$^5$ : **C08F 2/38**

(21) Anmeldenummer : **90124593.6**

(22) Anmeldetag : **18.12.90**

## (54) Verfahren zur kontinuierlichen Telomerisation.

(30) Priorität : **21.12.89 DE 3942316**

(43) Veröffentlichungstag der Anmeldung : **26.06.91 Patentblatt 91/26**

(45) Bekanntmachung des Hinweises auf die Patenterteilung : **10.11.93 Patentblatt 93/45**

(84) Benannte Vertragsstaaten : **DE FR GB IT NL**

(56) Entgegenhaltungen : **DE-A- 1 443 517**

(73) Patentinhaber : **HOECHST AKTIENGESELLSCHAFT**
**D-65926 Frankfurt (DE)**

(72) Erfinder : **Paul, Norbert**
**Kiem-Pauli-Strasse 8a**
**W-8262 Altötting (DE)**
Erfinder : **Huber, Rudolf**
**Hütweg 12b**
**W-8268 Garching (DE)**
Erfinder : **Mielke, Ingolf, Dr.**
**Hochstaufenstrasse 16**
**W-8269 Burgkirchen (DE)**

EP 0 433 988 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur kontinuierlichen Telomerisation, wobei eine telogene Verbindung, die unter den Reaktionsbedingungen flüssig ist, mit mindestens einer eine ethylenische Doppelbindung enthaltenden taxogenen Verbindung mit 2 bis 4 C-Atomen bei 40 bis 150 °C unter einem Druck von 0,1 bis 3 MPa in Gegenwart von mindestens einem Katalysator in einem langgestreckten zylindrischen Reaktionsraum umgesetzt wird.

Als Telomerisation beziehungsweise Telomerisations-Reaktion wird ein Verfahren bezeichnet, bei dem nach dem derzeitigen Stand der Kenntnis eine telogene Verbindung AX unter Einwirkung von Licht oder Wärme in zwei Bruchstücke A und X zerfällt, an die sich Verbindungen mit polymerisationsfähiger, meist ethylenischer Doppelbindung, nachfolgend "taxogene Verbindungen" genannt, einfach oder mehrfach hintereinander anlagern, nach folgender Reaktionsgleichung:

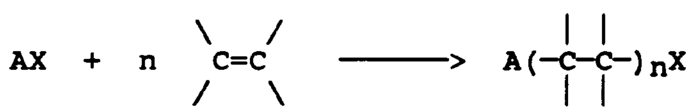

Es sind eine Anzahl Katalysatoren bekannt, die diese Reaktion beschleunigen, wodurch niedrigere Aktivierungstemperaturen ausreichen.

Unter den vielen möglichen Telomerisationsreaktionen, die mit taxogenen Verbindungen, die eine ethylenische Doppelbindung enthalten, möglich sind, haben solche wirtschaftliches Interesse erlangt, die zur Erzeugung kurz- und mittelkettiger, weitgehend fluorierter Verbindungen führen, wobei als telogene Verbindungen vorteilhaft Perfluoralkyliodide und als taxogene Verbindungen Ethylen oder weitgehend fluorierte Alkylene, wie Tetrafluorethen, Chlortrifluorethen oder Hexafluorpropen, verwendet werden.

Aus DE-AS 1 443 517 (C.A. Vol. 65, 1966, Nr. 20005f) ist ein Verfahren bekannt, bei dem Perfluoriodalkane als telogene Verbindungen gasförmig mit Tetrafluorethen oder Hexafluorpropen bei Temperaturen von 250 bis 800 °C und Drucken von 2 Torr bis 5 atm mit Verweilzeiten von weniger als 1 Stunde in einem rohrförmigen Reaktionsraum umgesetzt werden. Aus dem Gasgemisch werden die erwünschten telomeren Verbindungen kondensiert und das nicht kondensierte Gasgemisch nach Ergänzung der telogenen und der taxogenen Verbindung(en) wieder im Kreislauf zurückgeführt. Dieses Verfahren ergibt insbesondere bei niedrigen Drucken keine guten Raum-Zeit-Ausbeuten, ferner ist die Anwendung der genannten Temperaturen, insbesondere bei Drucken über dem normalen Atmosphärendruck, bei thermisch instabilen taxogenen Verbindungen, wie Tetrafluorethen, mit erheblichen Gefahren für einen spontanen unkontrollierbaren Zerfall verbunden.

Wie weiter oben bereits erwähnt, sind für die Telomerisationsreaktion eine ganze Anzahl Katalysatoren bekannt, wobei in den Beispielen der entsprechenden Schriften in der Regel diskontinuierliche Verfahren (Kochen am Rückfluß mit Lösungsmitteln, einmalige beziehungsweise absatzweise Beschickung eines Autoklaven) angewendet werden. Stellvertretend für eine Reihe ähnlicher Schriften sei hier das in GB-PS 1 535 408 beschriebene Verfahren zur Herstellung von Perfluoriodidtelomeren durch Umsetzung von Perfluoralkyliodiden mit Tetrafluorethen bei erhöhtem Druck und erhöhter Temperatur in flüssiger Phase in Gegenwart von Di-(alkylphenyl)-peroxydicarbonaten als radikalbildende Katalysatoren erwähnt, bei dem nach der Reaktion die homologen Perfluoralkyliodide mit 1 bis 5 C-Atomen durch fraktionierte Destillation abgetrennt und in den Prozeß zurückgeführt werden. Das Verfahren läuft bei Drucken von 5 bis 17 atü und bei Temperaturen von 45 bis 100 °C ab und kann diskontinuierlich, halbkontinuierlich (mit diskontinuierlicher Entnahme des Reaktionsgemisches) sowie auch vollkontinuierlich durchgeführt werden. Genauere Angaben zur vollkontinuierlichen Fahrweise sind nicht enthalten, die Beispiele werden ausschließlich diskontinuierlich durchgeführt.

Gegenüber dem aus DE-AS 1 443 517 (C.A. Vol. 65, 1966, 20005f) bekannten Verfahren ergibt sich der wesentliche Vorteil, daß bei tieferer Temperatur in flüssiger Phase gearbeitet werden kann, wodurch die Gefahr spontaner Zersetzung von thermisch instabilen taxogenen Verbindungen, wie Tetrafluorethen, wesentlich vermindert wird, jedoch lassen bei der rationelleren, kontinuierlichen Fahrweise in einem langgestreckten, zylindrischen Reaktionsraum die Raum-Zeit-Ausbeuten insbesondere mittelkettiger Telomerisate zu wünschen übrig, wie nachfolgende Vergleichsversuche zeigen.

Es wurde nun ein Verfahren gefunden, das günstigere Raum-Zeit-Ausbeuten ermöglicht und eine bessere Steuerung der Reaktion im Hinblick auf Produkte bestimmter Zusammensetzung, insbesondere bestimmter C-Kettenlänge, ermöglicht.

Das neue Verfahren zur kontinuierlichen Telomerisation von mindestens einer telogenen Verbindung, die unter den Reaktionsbedingungen flüssig ist, mit mindestens einer eine ethylenische Doppelbindung enthaltenden taxogenen Verbindung mit 2 bis 4 C-Atomen bei einer Temperatur von 40 bis 150 °C und einem Druck von 0,1 bis 3 MPa in Gegenwart mindestens eines Katalysators in einem langgestreckten zylindrischen Reaktionsraum, nach Beendigung der Telomerisationsreaktion und Verlassen des Reaktionsraumes Auftrennung der Reaktionsmischung und Abführung der erwünschten höhermolekularen Telomerisationsprodukte sowie Rückführung und Wiedereinsetzung zur Telomerisationsreaktion der unerwünschten niedriger-molekularen Telomerisationsprodukte und der nicht umgesetzten Ausgangssubstanzen in einem ersten Kreislauf, wobei die verbrauchten Ausgangssubstanzen ergänzt werden, ist dadurch gekennzeichnet, daß ein Teil der im wesentlichen flüssigen Reaktionsmischung aus der in Strömungsrichtung zweiten Hälfte des Reaktionsraumes abgeführt und ohne Abtrennung von Bestandteilen in einem zweiten Kreislauf in die in Strömungsrichtung erste Hälfte des Reaktionsraumes wieder eingeführt wird, wobei zwischen Abführung und Einführung 20 bis 90 % der Gesamtlänge des Reaktionsraumes liegen.

Als telogene Verbindungen, die unter den Reaktionsbedingungen flüssig sind, sind eine ganze Reihe Verbindungen geeignet, beispielsweise Alkanole mit 1 bis 4 C-Atomen, insbesondere, wenn als taxogene Verbindungen weitgehend fluorierte ethylenische Verbindungen zum Einsatz kommen. Vorzugsweise werden als telogene Verbindungen weitgehend fluorierte Alkyliodide der Formel

$$R\text{-}(CF_2)_n\text{-}I$$

eingesetzt, in der bedeuten:
R gleich H; F oder

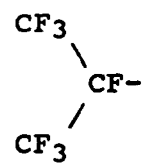

und n eine Zahl von 1 bis 6, wobei bevorzugt telomere Verbindungen eingesetzt werden, die nicht mehr als 6 C-Atome enthalten. In vielen Fällen werden Mischungen verschiedener telomerer Verbindungen verwendet.

Aus der Vielzahl geeigneter taxogener Verbindungen, die eine ethylenische Doppelbindung und 2 bis 4 C-Atome enthalten, werden vorzugsweise eingesetzt: Ethylen, Vinylfluorid, Vinylidenfluorid, Chlortrifluorethylen und insbesondere Hexafluorpropen und Tetrafluorethen.

Das erfindungsgemäße Verfahren wird bei einer Temperatur von 40 bis 150 °C durchgeführt. Unter 40 °C läuft die Reaktion auch unter Zusatz von Katalysatoren im allgemeinen zu langsam, Temperaturen über 150 °C sind zwar möglich, jedoch im allgemeinen nicht erforderlich, sie stellen außerdem ein zunehmendes Sicherheitsrisiko und einen im allgemeinen unnötigen zusätzlichen Aufwand dar. Vorzugsweise wird bei Temperaturen von 50 bis 100 °C gearbeitet.

Das neue Verfahren läuft bei einem Druck von 0,1 bis 3 MPa. Prinzipiell ist auch die Anwendung eines Druckes unter 0,1 MPa möglich, doch ergibt diese unnötig ungünstige Raum-Zeit-Ausbeuten. Der anzuwendende maximale Druck hängt von der Stabilität und Reaktivität insbesondere der taxogenen Verbindungen ab, wobei in der Regel ein Druck über 3 MPa nicht erforderlich ist. Vorzugsweise wird bei einem Druck von 0,3 bis 2,5 MPa gearbeitet. Bei der Anwendung von Tetrafluorethen als taxogener Verbindung sollte zweckmäßig ein Maximaldruck von 2,5 MPa aus Sicherheitsgründen nicht überschritten werden.

Die Telomerisationsreaktion wird in Gegenwart mindestens eines Katalysators vorgenommen. Geeignete Katalysatoren sind radikalisch zerfallende peroxidische Verbindungen, die bei der gewählten Reaktionstemperatur eine Halbwert-Zerfallszeit im Bereich von etwa 3 bis 60 Minuten aufweisen, sowie auch Metallsalze für sich, in Mischung miteinander sowie in Mischung mit Aminverbindungen als Metallion-Aminsysteme. Als radikalisch zerfallende peroxidische Verbindungen seien beispielsweise genannt Peroxyalkancarbonsäuren, deren Wasserstoffatome ganz oder teilweise mit Fluor oder Chlor substituiert sein können, deren Anhydride und Ester, wie Tertiärbutylperpivalat, Peroxyalkohole, wie Ditertiärbutylhydroperoxid, oder -ether, wie Ditertiärbutylperoxid, sowie aliphatisch oder aromatisch substituierte Peroxydicarbonate. Geeignete Metallsalze sind beispielsweise die Halide, Phosphate, Carbonate, Nitrate, Sulfate, Cyanide, Hydride, Ethoxide von Metallen der Gruppen Ia, IIa, IIIa, IIIb bis VIb und VIIIb. Solche Metallsalze, insbesondere die Halide, werden zusammen mit primären, sekundären oder tertiären Alkyl-, Cycloalkyl-, Aryl- oder Alkanolaminen sowie auch heterocyclischen Aminen, beispielsweise Pyridin, verwendet, wobei auch Mischungen von Metallsalzen untereinander Anwendung finden. Es werden auch radikalisch zerfallende Azo-Verbindungen eingesetzt, beispielsweise Azoisobuttersäuredinitril. Die Menge des Katalysators, bezogen auf die eingesetzte Menge telogener Verbindung, beträgt im allgemeinen 0,05 bis 1 Gew.-%, vorzugsweise werden 0,1 bis 0,5 Gew.-%, eingesetzt.

Für das erfindungsgemäße Verfahren dient ein langgestreckter zylindrischer Reaktionsraum, zweckmäßig ein Rohr, der beziehungsweise das vorzugsweise ein Verhältnis von Länge zu innerem Durchmesser von 500

bis 20 000, insbesondere von 1 000 bis 10 000 aufweist. Der freie Durchtrittsquerschnitt des Reaktionsraumes ist nicht kritisch, im allgemeinen wird man solche Reaktionsräume (Rohre) verwenden, die einen freien Durchtrittsquerschnitt von etwa 1 bis etwa 50 $cm^2$ aufweisen. Ebenfalls unkritisch ist die Anordnung des Reaktionsraumes; er kann senkrecht, waagrecht oder in einem bestimmten Winkel zur Waagrechten geneigt angeordnet sein, als Rohr in übereinanderliegenden Windungen oder zickzackförmig vorliegen.

Bei einer Ausführungsform der Erfindung wird die taxogene Verbindung in den Anfang des Reaktionsraumes eingespeist; die unter den Reaktionsbedingungen flüssige(n) telogene(n) Verbindung(en) wird (werden) in einem Anstand von 0 bis 25 % der Gesamtlänge des Reaktionsraumes vom Anfang dieses Reaktionsraumes entfernt eingespeist, wobei diese Einspeisung vorteilhaft nach der Wiedereinführung der erfindungsgemäß im Kreis laufenden Reaktionsmischung (zweiter Kreislauf) erfolgt. Die telogene(n) Verbindung(en) kann (können) vor dem Eintritt in den Reaktionsraum temperiert werden, beispielsweise um sie in flüssigen Zustand überzuführen. Die telogenen Verbindungen können an verschiedenen Stellen des Reaktionsraumes eingespeist werden, im allgemeinen ist jedoch eine Einspeisung an einer Stelle ausreichend. Der Katalysator wird zweckmäßig zusammen mit den telogenen Verbindungen in diesen gelöst oder suspendiert in den Reaktionsraum eingeführt.

Nach Verlassen des Reaktionsraumes wird die Reaktionsmischung aufgetrennt. Dies kann zweckmäßig durch Destillation unter erhöhtem Druck, normalem Atmosphärendruck oder vermindertem Druck geschehen. Zwischen dem Reaktionsraum und der Destillationseinrichtung ist zweckmäßig eine Vorrichtung, beispielsweise ein Druckminderventil, angeordnet, die eine Druckdifferenz aufrechtzuerhalten gestattet. Der weiter oben angegebene Druck im Reaktionsraum ist der dort herrschende Maximaldruck. Er wird in der Regel am Anfang des Reaktionsraumes gemessen. Die Reaktionsmischung wird in die erwünschten, in der Regel höher siedenden Telomerisationsprodukte, die bei der Destillation im Sumpf verbleiben, und die nicht erwünschten, niedriger siedenden Telomerisationsprodukte sowie die unveränderten Ausgangssubstanzen, die bei der Destillation am Kopf übergehen, aufgetrennt. Sofern eine Destillation angewendet wird, wird der Druck im Destillationsapparat möglichst so gewählt, daß sich eine Sumpftemperatur von 50 bis 150 °C einstellt. Sumpftemperaturen über etwa 170 °C sollten nicht überschritten werden, da höhere Temperaturen häufig zu unerwünschter Zersetzung der Telomerisate führen. Das Sumpfprodukt wird gekühlt und in einem Vorratsbehälter gesammelt. Obwohl bei der Telomerisation in der Regel Gemische verschiedener Verbindungen erhalten werden, ist für viele Anwendungszwecke das Sumpfprodukt ohne weitere Reinigung brauchbar. Falls erforderlich, kann es in seine Komponenten nach bekannten Methoden weiter aufgetrennt werden.

Die von den erwünschten Telomerisaten abgetrennten unerwünschten (niedriger-molekularen) Telomerisate und unveränderten Ausgangsprodukte werden zweckmäßig gekühlt, wobei die niedrig-molekularen Telomerisate und die als Ausgangsprodukte verwendete(n) telogene(n) Verbindung(en) kondensieren. Diesem Kondensat werden zweckmäßig die Menge der zur Erzeugung der erwünschten Telomerisate verbrauchten telogenen Verbindung(en) sowie der Katalysator zugesetzt und diese Flüssigkeit in einem ersten Kreislauf in den Reaktionsraum, wie oben beschrieben, wieder eingespeist. Die bei der Kondensation der nicht erwünschten Telomerisate nicht kondensierbaren Verbindungen werden gegebenenfalls nach Reinigung und/oder Abtrennung und Wiederverwendung der darin enthaltenen taxogenen Verbindung(en) als Abgas in die Atmosphäre entlassen.

In den Reaktionsraum wird (werden) mindestens soviel taxogene Verbindung(en) eingespeist, wie aufgrund der Stöchiometrie zur Erzeugung der erwünschten Telomerisate notwendig ist (sind). Zweckmäßig wird ein geringer molarer Überschuß verwendet, wobei die eingespeiste Menge so bemessen wird, daß sich höchstens 10 % von ihr in den nicht kondensierbaren Anteilen der aufgetrennten Reaktionsmischung befinden.

Die dem ersten Kreislauf, der die abgetrennten, unerwünschten Telomerisate enthält, zugesetzte Menge neuer, unter den Reaktionsbedingungen flüssiger telogener Verbindung sollte zweckmäßig so bemessen werden, daß sich nach Einstellung konstanter Bedingungen im kontinuierlichen Verfahren eine rechnerische mittlere Verweilzeit im Reaktionsraum nur unter Berücksichtigung der flüssig zugeführten Verbindungen von 5 bis 50 Minuten ergibt. In diesem Bereich werden im allgemeinen gute Ergebnisse erhalten, vorzugsweise wird mit rechnerischen mittleren Verweilzeiten von 10 bis 30 Minuten gearbeitet.

Vorzugsweise beträgt das Verhältnis der im ersten Kreislauf rückgeführten Produktmenge zu der Summe der Mengen der neueingespeisten Ausgangssubstanzen für die Telomerisations-reaktion 10 bis 50.

Erfindungsgemäß wird ein Teil der im wesentlichen flüssigen Reaktionsmischung aus der in Strömungsrichtung zweiten Hälfte des Reaktionsraumes abgeführt und ohne Abtrennung von Bestandteilen in einem zweiten Kreislauf in die in Strömungsrichtung erste Hälfte des Reaktionsraumes wieder eingeführt. Die Abnahmestelle ist zweckmäßig mindestens 10 und bis zu 45 %, vorzugsweise 20 bis 40 % der Gesamtlänge des Reaktionsraumes von dessen Ende entfernt und die Wiederzuführungsstelle 0 bis 35 %, vorzugsweise 0 bis 10 %, der Gesamtlänge des Reaktionsraumes von dessen Anfang entfernt liegen. Der Abstand von der Abnahme zur Wiederzuführungsstelle beträgt 20 bis 90 % der Gesamtlänge des Reaktionsraumes. Ist der Abstand geringer als 20 %, ist der erfinderische Effekt nur noch gering. Bei einem Abstand über 90 % tritt häufig eine zu ungünstige Ausnützung des Katalysators ein. Vorzugsweise wird ein Abstand von 40 bis 80 % der Gesamtlänge des Reaktionsraumes eingehalten.

Der zweite Kreislauf wird zweckmäßig durch ein Rohr mit einer Pumpe bewerkstelligt, die die abgenommene Reaktionsmischung zur Wiederzuführungsstelle fördert. Der freie Durchtrittsquerschnitt des Kreislaufrohres kann ebenso groß sein wie der freie Durchtrittsquerschnitt des Reaktionsraumes oder etwas kleiner beziehungsweise etwas größer. Als Pumpe ist prinzipiell jede für die Förderung eines Gas-Flüssigkeits-Gemisches brauchbare Druckpumpe, beispielsweise eine Membran-, Kolben- oder Zahnradpumpe, geeignet.

Die Menge der im zweiten Kreislauf abgeführten und wieder eingespeisten Reaktionsmischung soll vorzugsweise das 0,5- bis 50fache der Summe der Mengen der im ersten Kreislauf wieder eingesetzten Produkte und der neu hinzugefügten Ausgangssubstanzen betragen. In diesem Bereich ist das erfindungsgemäße Verfahren gut durchführbar. Besonders gute Ergebnisse werden erhalten, wenn das 2- bis 20fache angewendet wird.

Gute Ergebnisse werden ferner erhalten, wenn das Verhältnis der im ersten Kreislauf als Kondensat aus dem Kopfprodukt der Destillation gewonnenen Produktmenge zu der Summe der Mengen der neu eingespeisten Ausgangssubstanzen für die Telomerisationsreaktion (telogene und taxogene Verbindung) 10 bis 50 beträgt.

Wie eingangs bereits erwähnt, ermöglicht das erfindungsgemäße Verfahren höhere Raum-Zeit-Ausbeuten und eine bessere Auswahl von gezielten Produktschnitten. Der apparative Aufwand zur Durchführung des erfindungsgemäßen Verfahrens ist gering.

Nachfolgende Vergleichsversuche und Beispiele sollen die Erfindung näher erläutern.

Vergleichsversuch A

Es wird folgende Apparatur verwendet:

Ein Behälter mit Rührer ist über eine Dosierpumpe mit einem Reaktionsrohr verbunden, das ein Verhältnis der Länge zum Innendurchmesser von 8 080 aufweist. Das eine Ende des Reaktionsrohres ist mit einer Einleitung eines unter Druck stehenden, als taxogene Verbindung dienenden Gases verbunden, vom anderen Ende des Reaktionsrohres führt eine Leitung mit einem Druckregulierventil zu einer Destillationskolonne, die eine Trennwirkung von 15 theoretischen Böden aufweist. Das Reaktionsrohr ist von einer Temperierflüssigkeit umströmt, die ihrerseits über einen Thermostaten und eine Pumpe im Kreis geführt ist. Der Sumpf der Destillationskolonne hat eine Öffnung zum Abführen des dort angesammelten Produktes, das über einen Produkt-Kühler in ein Produkt-Sammelgefäß läuft. Das Kopfprodukt der Destillationskolonne läuft über einen Destillatkühler, in dem es größtenteils kondensiert wird. Die nicht kondensierbaren Anteile werden über eine Kühlfalle in die Atmosphäre entlassen. Das Kondensat läuft in ein Kondensat-Sammelgefäß, das wiederum über einen Hahn mit dem eingangs genannten Behälter mit Rührer verbunden ist. Dieser Behälter ist temperierbar, hat etwa den 5fachen Inhalt des Reaktionsrohres und enthält je eine Einrichtung zur Zugabe von flüssiger telogener Ausgangsverbindung und von Katalysator.

Zur Durchführung des Vergleichsversuches wird in dem Behälter mit Rührer eine Lösung hergestellt, die zu Beginn des Versuches 0,018 Gew.-% eines peroxidisch zerfallenden Katalysators, der bei 90 °C eine Halbwerts-Zerfallszeit von 1,2 Minuten aufweist, in Perfluorethyliodid gelöst, enthält. 7,70 Gew.-Teile dieser Lösung werden je Stunde über die Dosierpumpe in das auf 92 °C temperierte Reaktionsrohr eingeführt, in das außerdem 0,20 Gew.-Teile je Stunde Tetrafluorethylen unter Druck eingeleitet werden. Der Druck am Beginn des Reaktionsrohres beträgt 1,4 MPa. Die Reaktionsmischung verläßt nach einer mittleren Verweilzeit von etwa 16 Minuten das Reaktionsrohr und wird über das Druckregulierventil in die Destillationskolonne eingespeist. Nachdem sich konstante Bedingungen eingestellt haben, beträgt die Sumpftemperatur 150 °C. Nun werden aus dem Sumpf der Destillationskolonne je Stunde 0,31 Gew.-Teile eines Produktes über den Kühler in das Produkt-Sammelgefäß abgeführt; dieses Produkt enthält aufgrund gaschromatographischer Analyse

| | | | |
|---|---|---|---|
| $C_6F_{13}I$ | 37,7 % | = | 0,117 Gew.-Teile/h |
| $C_8F_{17}I$ | 39,8 % | = | 0,123 Gew.-Teile/h |
| $C_{10}F_{21}I$ und höhere | 22,5 % | = | 0,070 Gew.-Teile/h |

Das Kopfprodukt der Destillationskolonne ergibt nach Kondensation im Destillatkühler 7,54 Gew.-Teile pro Stunde einer Flüssigkeit, die im wesentlichen das Ausgangsprodukt $C_2F_5I$ und niedrige Telomerisate $C_4F_9I$ und $C_6F_{13}I$ enthält. Diese Flüssigkeit wird in das Kondensat-Sammelgefäß und von dort in den Behälter mit Rührer

geleitet. Dort werden 0,15 Gew.-Teile/h $C_2F_5I$ und 0,0014 Gew.-Teile/h Katalysator zugesetzt, wobei wiederum eine 0,018 Gew.-% Katalysator enthaltende Lösung erhalten wird, die erneut in das Reaktionsrohr eingespeist wird. Aus den nicht kondensierbaren Anteilen aus dem Kopfprodukt der Destillationskolonne werden in der Kühlfalle 0,035 Gew.-Teile/h auskondensiert, das restliche Abgas wird in die Atmosphäre entlassen.

Beispiel 1

Es wird verfahren wie im Vergleichsversuch A angegeben, jedoch enthält das Reaktionsrohr in einem Abstand von 32,7 % der Gesamtlänge des Reaktionsrohres vom Ende dieses Rohres entfernt ein abzweigendes Rohr, das den gleichen Querschnitt wie das Reaktionsrohr aufweist und über eine Kreislaufpumpe mit regelbarer Leistung, die entgegen der Strömungsrichtung der Mischung im Reaktionsrohr fördert, mit dem Anfang des Reaktionsrohres verbunden ist. Der Abstand der Wiedereinführung des Abzweigrohres ist also 0 % der Gesamtlänge des Reaktionsrohres vom Anfang dieses Rohres entfernt. Anfang und Ende des Reaktionsrohres sind in Strömungsrichtung der Mischung in diesem Rohr gemeint. Die Leistung der Kreislaufpumpe wird so eingestellt, daß 63 Gew.-Teile/h der Reaktionsmischung umgepumpt werden. Zwischen Abführung aus dem und Wiedereinführung des umgepumpten Teils der Mischung in das Reaktionsrohr liegen 67,3 % der Gesamtlänge des Reaktionsrohres. Das Verhältnis der im ersten Kreislauf rückgeführten Produktmenge (7,54 Gew.-Teile/h) zu der Summe der Menge der neu eingespeisten Ausgangssubstanzen (0,20 + 0,15 = 0,35 Gew.-Teile/h) beträgt 21,5. Die im zweiten Kreislauf abgeführte und wieder eingespeiste Menge der Reaktionsmischung (63 Gew.-Teile/h) beträgt das 8fache von der in das Reaktionsrohr eingeführten Mischung aus wieder eingesetzten Produkten (7,54 Gew.-Teile/h) und neu hinzugefügten Ausgangssubstanzen (0,35 Gew.-Teile/h).

Nach Einstellung konstanter Bedingungen beträgt der Druck am Beginn des Reaktionsrohres 1,75 MPa und die Sumpftemperatur der Destillationskolonne 158 °C. Im Produkt-Sammelgefäß werden 0,32 Gew.-Teile/h eines Produktes erhalten, welches nach gaschromatographischer Analyse enthält:

| | | | |
|---|---|---|---|
| $C_6F_{13}I$ | 21,7 % | = | 0,069 Gew.-Teile/h |
| $C_8F_{17}I$ | 50,0 % | = | 0,160 Gew.-Teile/h |
| $C_{10}F_{21}I$ und höhere | 28,3 % | = | 0,091 Gew.-Teile/h |

Aus den im Destillatkühler nicht kondensierbaren Anteilen aus dem Kopfprodukt der Destillationskolonne werden 0,023 Gew.-Teile/h auskondensiert, das restliche Abgas wird in die Atmosphäre entlassen.

Wie ersichtlich, wird gegenüber Vergleichsversuch A, unter gleichen Bedingungen, ein um 30,1 % höherer Anteil der erwünschten Telomerisate mit 8 und mehr C-Atomen erhalten bei verringerter Abgasmenge. Die Steigerung des Anteils an $C_8F_{17}I$ beträgt 30,1 %.

Vergleichsversuch B

In der gleichen Apparatur wie im Vergleichsversuch A beschrieben, werden 7,70 Gew.-Teile/h der Katalysatorlösung, die 0,018 Gew.-% eines peroxidisch zerfallenden Katalysators, der bei 90 °C eine Halbwerts-Zerfallszeit von 1,2 Minuten aufweist, enthält, über die Dosierpumpe in das auf 94 °C temperierte Reaktionsrohr eingeführt, in das außerdem 0,29 Gew.-Teile/h Tetrafluorethylen unter Druck eingeleitet werden. Der Druck am Beginn des Reaktionsrohres beträgt 1,4 MPa. Die Reaktionsmischung verläßt nach einer mittleren Verweilzeit von etwa 16 Minuten das Reaktionsrohr und wird über das Druckregulierventil in die Destillationskolonne eingespeist. Nachdem sich konstante Bedingungen eingestellt haben, beträgt die Sumpftemperatur 155 °C. Nun werden aus dem Sumpf der Destillationskolonne 0,40 Gew.-Teile/h eines Produktes über den Kühler in das Produkt-Sammelgefäß abgeführt; dieses Produkt enthält aufgrund gaschromatographischer Analyse

| | | | |
|---|---|---|---|
| $C_6F_{13}I$ | 36,2 % | = | 0,145 Gew.-Teile/h |
| $C_8F_{17}I$ | 37,5 % | = | 0,150 Gew.-Teile/h |
| $C_{10}F_{21}I$ und höhere | 26,3 % | = | 0,105 Gew.-Teile/h |

Das Kopfprodukt der Destillationskolonne ergibt nach Kondensation im Destillatkühler 7,51 Gew.-Teile pro Stunde einer Flüssigkeit, die im wesentlichen das Ausgangsprodukt $C_2F_5I$ und niedrige Telomerisate $C_4F_9I$ und $C_6F_{13}I$ enthält. Diese Flüssigkeit wird in das Kondensat-Sammelgefäß und von dort in den Behälter mit Rührer

geleitet. Dort werden 0,18 Gew.-Teile/h $C_2F_5I$ und 0,0014 Gew.-Teile/h Katalysator zugesetzt, wobei wiederum eine 0,018 Gew.-% Katalysator enthaltende Lösung erhalten wird, die erneut in das Reaktionsrohr eingespeist wird. Aus den nicht kondensierbaren Anteilen aus dem Kopfprodukt der Destillationskolonne werden in der Kühlfalle 0,074 Gew.-Teile/h auskondensiert, das restliche Abgas wird in die Atmosphäre entlassen.

Beispiel 2

Es wird verfahren wie im Vergleichsversuch B angegeben, wobei das in Beispiel 1 beschriebene Abzweigrohr mit Kreislaufpumpe am Reaktionsrohr verwendet wird. Die Leistung der Kreislaufpumpe wird so eingestellt, daß 33,6 Gew.-Teile/h der Reaktionsmischung umgepumpt werden. Nach Einstellung konstanter Bedingungen beträgt der Druck am Beginn des Reaktionsrohres 1,6 MPa und die Sumpftemperatur der Destillationskolonne 153 °C. Im Produkt-Sammelgefäß werden 0,505 Gew.-Teile/h eines Produktes erhalten, welches nach gaschromatographischer Analyse enthält:

| | | | |
|---|---|---|---|
| $C_6F_{13}I$ | 37,6 % | = | 0,190 Gew.-Teile/h |
| $C_8F_{17}I$ | 37,0 % | = | 0,187 Gew.-Teile/h |
| $C_{10}F_{21}I$ und höhere | 25,4 % | = | 0,128 Gew.-Teile/h |

Aus den im Destillatkühler nicht kondensierbaren Anteilen aus dem Kopfprodukt der Destillationskolonne werden 0,019 Gew.-Teile/h auskondensiert, das restliche Abgas wird in die Atmosphäre entlassen. Das Verhältnis der nach der Destillation im ersten Kreislauf zurückgeführten, wieder eingesetzten Produktmenge (7,47 Gew.-Teile/h) zur Summe der Mengen der neu eingespeisten Ausgangssubstanzen (0,29 + 0,23 = 0,52 Gew.-Teile/h) beträgt 14,4. Die im zweiten Kreislauf abgeführte und wieder eingespeiste Menge der Reaktionsmischung (33,6 Gew.-Teile/h) beträgt das 4,2fache von der in das Reaktionsrohr eingeführten Mischung aus wieder eingesetzten Produkten (7,47 Gew.-Teile/h) und neu hinzugefügten Ausgangssubstanzen (0,52 Gew.-Teile/h).

Wie ersichtlich, wird gegenüber Vergleichsversuch B, unter gleichen Bedingungen, ein um 23,5 % höherer Anteil der erwünschten Telomerisate mit 8 und mehr C-Atomen erhalten bei verminderter Abgasmenge. Die Steigerung des Anteils an $C_8F_{17}I$ beträgt 24,7 %.

**Patentansprüche**

1. Verfahren zur kontinuierlichen Telomerisation von mindestens einer telogenen Verbindung, die unter den Reaktionsbedingungen flüssig ist, mit mindestens einer eine ethylenische Doppelbindung enthaltenden taxogenen Verbindung mit 2 bis 4 C-Atomen bei einer Temperatur von 40 bis 150 °C und einem Druck von 0,1 bis 3 MPa in Gegenwart mindestens eines Katalysators in einem langgestreckten zylindrischen Reaktionsraum, nach Beendigung der Telomerisationsreaktion und Verlassen des Reaktionsraumes Auftrennung der Reaktionsmischung und Abführung der erwünschten höhermolekularen Telomerisationsprodukte sowie Rückführung und Wiedereinsetzung zur Telomerisationsreaktion der unerwünschten niedriger-molekularen Telomerisationsprodukte und der nicht umgesetzten Ausgangssubstanzen in einem ersten Kreislauf, wobei die verbrauchten Ausgangssubstanzen ergänzt werden, dadurch gekennzeichnet, daß ein Teil der im wesentlichen flüssigen Reaktionsmischung aus der in Strömungsrichtung zweiten Hälfte des Reaktionsraumes abgeführt und ohne Abtrennung von Bestandteilen in einem zweiten Kreislauf in die in Strömungsrichtung erste Hälfte des Reaktionsraumes wieder eingeführt wird, wobei zwischen Abführung und Einführung 20 bis 90 % der Gesamtlänge des Reaktionsraumes liegen.

2. Verfahren nach Anspruch 1, dadurch gekennzeichnet, daß die im zweiten Kreislauf abgeführte und wieder eingespeiste Menge der Reaktionsmischung das 0,5- bis 50fache der Summe der Mengen der im ersten Kreislauf wieder eingesetzten Produkte und der neu hinzugefügten Ausgangssubstanzen beträgt.

3. Verfahren nach Anspruch 1 oder 2, dadurch gekennzeichnet, daß der langgestreckte zylindrische Reaktionsraum ein Verhältnis von Länge zu Durchmesser von 500 bis 20 000 aufweist.

4. Verfahren nach einem oder mehreren der Ansprüche 1 bis 3, dadurch gekennzeichnet, daß das Verhältnis der im ersten Kreislauf rückgeführten Produktmenge zu der Summe der Mengen der neu eingespeisten

Ausgangssubstanzen für die Telomerisationsreaktion 10 bis 50 beträgt.

5. Verfahren nach einem oder mehreren der Ansprüche 1 bis 4, dadurch gekennzeichnet, daß bei einem Druck von 0,3 bis 2,5 MPa gearbeitet wird.

## Claims

1. A process for continuous telomerization of at least one telogenic compound, which is liquid under the reaction conditions, with at least one taxogenic compound having an ethylenic double bond and 2 to 4 carbon atoms, at a temperature of from 40 to 150°C and under a pressure of from 0.1 to 3 MPa in the presence of at least one catalyst in an elongate cylindrical reaction space, separation of the reaction mixture after the telomerization reaction has ended and the mixture has left the reaction space, and discharge of the desired higher-molecular telomerization products as well as recycle of the undesired lower-molecular telomerization products and of the unconverted starting substances and reintroduction thereof to the telomerization reaction in a first circulation, the consumed starting substances being made up, which comprises discharging a part of the essentially liquid reaction mixture from that half of the reaction space which is the second in the direction of flow and reintroducing it, without separating off constituents, in a second circulation into that half of the reaction space which is the first in the direction of flow, 20 to 90 % of the total length of the reaction space being located between the discharge and the introduction.

2. The process as claimed in claim 1, wherein the rate of the reaction mixture discharged and fed back in the second circulation is 0.5 to 50 times the total of the rates of the products reintroduced in the first circulation and of the newly added starting substances.

3. The process as claimed in claim 1 or 2, wherein the elongate cylindrical reaction space has a length/diameter ratio of from 500 to 20,000.

4. The process as claimed in one or more of claims 1 to 3, wherein the ratio of the product rate recycled in the first circulation to the total of the rates of the newly fed starting substances for the telomerization reaction is 10 to 50.

5. The process as claimed in one or more of claims 1 to 4, which is operated under a pressure of from 0.3 to 2.5 MPa.

## Revendications

1. Procédé pour la télomérisation en continu d'au moins un composé télogène, qui est liquide dans les conditions de réaction, avec au moins un composé taxogène contenant une double liaison éthylénique et ayant 2 à 4 atomes de carbone, à une température de 40 à 150°C et sous une pression de 0,1 à 3 MPa en présence d'au moins un catalyseur dans un espace cylindrique allongé de réaction, après achèvement de la réaction de télomérisation et après que le mélange réactionnel a quitté l'espace de réaction, séparation du mélange réactionnel et soutirage des produits voulus de télomérisation, à poids moléculaire élevé, ainsi que recyclage et réutilisation, pour la réaction de télomérisation, des produits non souhaités de télomérisation à bas poids moléculaire et des substances de départ n'ayant pas réagi, dans un premier circuit, avec remplacement des substances de départ consommées, procédé caractérisé en ce qu'on soutire de la seconde moitié de l'espace de réaction (dans le sens de l'écoulement) une partie du mélange réactionnel, essentiellement liquide, et on l'introduit, sans en séparer les constituants, dans un second circuit dans la première moitié (dans le sens de l'écoulement) de l'espace de réaction, une proportion de 20 à 90 % de la longueur totale de l'espace de réaction se situant entre le point de soutirage ou de prélèvement et le point d'introduction.

2. Procédé selon la revendication 1, caractérisé en ce que la quantité du mélange réactionnel soutirée du second circuit et réintroduite représente 0,5 à 50 fois la somme des quantités des produits réutilisés dans le premier circuit et des substances de départ introduites à nouveau.

3. Procédé selon la revendication 1 ou 2, caractérisé en ce que l'espace cylindrique allongé de réaction présente un rapport, entre sa longueur et son diamètre, allant de 500 à 20 000.

4. Procédé selon une ou plusieurs des revendications 1 à 3, caractérisé en ce que le rapport de la quantité de produit recyclé dans le premier circuit et la somme des quantités des substances de départ introduites à nouveau pour la réaction de télomérisation vaut de 10 à 50.

5. Procédé selon une ou plusieurs des revendications 1 à 4, caractérisé en ce qu'on travaille sous une pression de 0,3 à 2,5 MPa.